Europäisches Patentamt

European Patent Office

Office européen des brevets

(19)

(11) Veröffentlichungsnummer : **0 048 681**
**B2**

(12) **NEUE EUROPÄISCHE PATENTSCHRIFT**

(45) Veröffentlichungstag der neuen Patentschrift :
15.04.87

(21) Anmeldenummer : 81710047.2

(22) Anmeldetag : 18.09.81

(51) Int. Cl.⁴ : **A 61 K   6/06**

(54) Verfahren zur Herstellung eines Verbundharzes, vorzugsweise zur Verwendung als zahnärztliches Material.

(30) Priorität : 19.09.80 SE 8006576

(43) Veröffentlichungstag der Anmeldung :
31.03.82 Patentblatt 82/13

(45) Bekanntmachung des Hinweises auf die Patenterteilung : 09.05.84 Patentblatt 84/19

(45) Bekanntmachung des Hinweises auf die Entscheidung über den Einspruch : 15.04.87 Patentblatt 87/16

(84) Benannte Vertragsstaaten :
CH DE FR GB LI SE

(56) Entgegenhaltungen :
EP-A- 0 026 398
DE-A- 2 405 578
DE-A- 2 446 546
DE-A- 2 711 014
DE-B- 2 403 211
DE-B- 2 724 814
US-A- 3 066 112
US-A- 3 709 866
US-A- 4 215 033
SCAND. J. DENT. RES., Band 88, 1980 L. EHRNFORD et al. "Bone Tissue Formation within a Sintered Microporous Glass-Fiber Network Implanted in Extraction Sockets in the Rat" Seiten 130 bis 133

(73) Patentinhaber : Ehrnford, Lars Edgar Martin
Sanekullavägen 31
S-217 74 Malmö (SE)

(72) Erfinder : Ehrnford, Lars Edgar Martin
Sanekullavägen 31
S-217 74 Malmö (SE)

(74) Vertreter : Patentanwälte, Dipl.-Ing. Klaus Westphal Dr. rer. nat. Bernd Mussgnug Dr. rer. nat. Otto Buchner
Flossmannstrasse 30a
D-8000 München 60 (DE)

EP 0 048 681 B2

Jouve, 18, rue St-Denis, 75001 Paris, France

## Beschreibung

Die Erfindung betrifft ein Verfahren zur Herstellung von Verbundharzen, vorzugsweise zur Verwendung als zahnärztliches Material, einschliesslich Füll- und Verkleidungsmaterial, Bindemittel, Material für Kronen und Brücken, sowie Material für künstliche Gebisse und Zähne.

Zur Herstellung von Verbundmaterialien ist ein Verfahren bekannt, bei dem Keramikteilchen mit einem Harz gebunden werden. Im allgemeinen wird von einer pastigen oder kittartigen Mischung eines härtbaren Monomers (organisches Flüssigharz) und festen Keramikteilchen ausgegangen. Die Keramikteilchen werden beim Aushärten des Monomers verklebt. Dieses Verfahren erfordert eine überschüssige Menge des Monomers, was dazu führt, dass die Teilchen in der ausgehärteten Struktur in einem wesentlichen Abstand voneinander angeordnet sind. Die Harzkomponente wirkt sich daher negativ auf die Eigenschaften des Verbundmaterials aus, mindestens im Hinblick auf die Festigkeit, Steifheit und Oberflächenstruktur. Weitere Eigenschaften wie Wärmeverhalten und viskoelastische Eigenschaften, Farbbeständigkeit, Oberflächenglätte und Schwund beim Härten werden dadurch ebenfalls beeinträchtigt. Ein derartiges Verbundharz für Zahnausbesserungen, mit einer Mischung eines flüssigen polymerisierbaren organischen Bindemittels und eines festen anorganischen Füllmittels ist in der US-PS 3 066 112 beschrieben.

Der Erfindung liegt die Aufgabe zugrunde, diese Nachteile der herkömmlichen Verfahren auszuschalten und ein Verfahren und eine Zusammensetzung zu schaffen, welche die Eigenschaften von Verbundharzen in dieser Beziehung wesentlich verbessert.

Gegenstand der Erfindung ist daher ein Verfahren zur Herstellung einer Verbundmasse aus organischem Harz und anorganischen porösen Teilchen, vorzugsweise zur Verwendung als zahnärztliches Ausbesserungsmaterial, dadurch gekennzeichnet, dass poröse anorganische Teilchen mit mindestens teilweise aushärtbarem Harzmaterial imprägniert, zusammengepresst oder auf andere Weise einem Druck dergestalt unterworfen werden, dass die Teilchen sich gegenseitig berühren und der Druck im Harz durch plastisches Fliessen des überschüssigen Harzes durch die Poren der anorganischen Teilchen ausgeglichen wird, und dass die Teilchen durch Aushärten mindestens eines Teils des aushärtbaren Harzmaterials zusammengeklebt werden und dadurch eine Harzstruktur mit einer angrenzenden, mit Harz imprägnierten anorganischen Phase bilden, wobei die porösen anorganischen Teilchen unter Ausschluß aller anderen porösen Füllstoffe ein starres dreidimensionales Netz von anorganischen Fasern enthalten, die zusammengeschweisst sind durch Erhitzen auf eine ausreichende Temperatur, um das Schmelzen und vollständige Zusammenschweissen im wesentlichen aller Einzelfasern an ihren Berührungspunkten zu einem Netz zu bewirken, in dem die Porosität durchgehend durch den ganzen Teil an den Berührungspunkten verläuft, an denen die Fasern gegeneinander anliegen.

Das nach diesem Verfahren hergestellte Verbundharz bietet auch vorteilhafte Verarbeitungseigenschaften bei der Durchführung von zahnärztlichen Ausbesserungen.

Desgleichen gestattet das Verfahren gemäss der Erfindung die Verwendung von Harzen mit besseren Eigenschaften als die bisher verfügbaren, insbesondere da nunmehr Harze mit einem höheren durchschnittlichen Molekulargewicht und höherer Viskosität verwendet werden können. Es ist möglich, ein Verbundmaterial mit weniger Harz zwischen den Teilchen zu erzielen als dies bisher bekannt war.

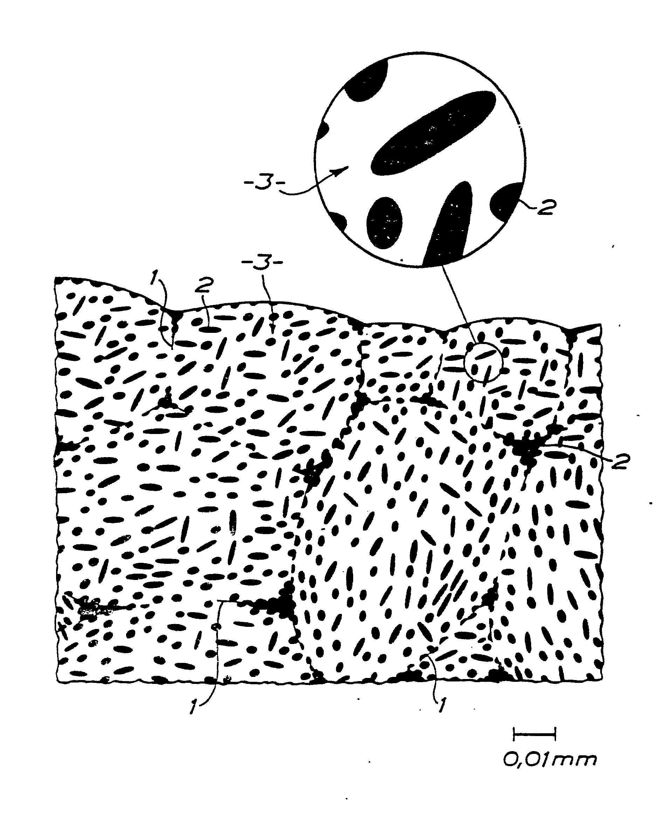

Anhand der Figur, die in eintausendfacher Vergrösserung einen Teil eines mit dem erfindungsgemässen Verfahren hergestellten Verbundharzes zeigt, wird ein Ausführungsbeispiel der Erfindung näher erläutert.

Das in der Figur dargestellte Verbundharz wurde nach dem Verfahren gemäss der Erfindung hergestellt, wobei eine Masse von porösen anorganischen Teilchen 1 vollständig oder teilweise mit mindestens zum Teil härtbarem Harzmaterial 2 dergestalt imprägniert wird, dass die Teilchen sich gegenseitig berühren und beim Härten mindestens eines Teils des härtbaren Harzmaterials zusammengeklebt werden und dadurch eine Harzstruktur mit einer angrenzenden anorganischen Phase bilden.

Bei dem Verfahren gemäss der Erfindung werden Teilchen verwendet, die ein starres dreidimensionales Netz von anorganischen Fasern enthalten, die an denjenigen Punkten zusammengesintert sind, an denen die Fasern sich gegenseitig berühren. Es ist besonders vorteilhaft, wenn die Fasern einen Durchmesser von weniger als 4 Mikrometer, vorzugsweise einen Durchmesser von 1 bis 3 Mikrometer aufweisen. Die Fasern mit extrem kleinem Durchmesser ergeben eine Struktur, die nach Abnützung eine äusserst glatte Oberfläche aufweist, was für zahnärztliche Ausbesserungen besonders vorteilhaft ist. Die anorganischen Fasern mit den obengenannten Abmessungen werden miteinander verschmolzen durch Erhitzen auf eine ausreichende Temperatur, die das Erweichen und vollständige Verschmelzen im wesentlichen aller einzelnen Fasern an ihren Berührungspunkten zu einem Netz bewirkt, in dem die Porosität durch das gesamte Netz durchgängig ist. Die Porosität eines solchen Netzes ist umfassend, und die meisten Porendurchmesser sind kleiner als 10 Mikrometer, und vorzugsweise ergibt das Verfahren ein Netz mit Porendurchmessern von weniger als 2 Mikrometer. Bezüglich der Oberflächenglätte ist es besonders vorteilhaft, wenn im wesentlichen alle Porendurchmesser kleiner als 10 Mikrometer und vorzugsweise kleiner als 2 Mikrometer sind. Ein derartiges Netz enthält vorzugsweise Glas, das einen hohen Grad von Undurchlässigkeit für Röntgenstrahlen aufweist und auch vorzugsweise aus äusserst feinen Fasern hergestellt ist, wie sie z. B. im nachfolgenden Beispiel verwendet werden :

2

Zwei quadratische und planparallele Pressplatten (12 × 12 × 1,5 cm) wurden aus einem hydraulischen Zement (Secar® 250, Lafarge Aluminous Cement Co. Ltd. Essex, England) in Edelstahlformen hergestellt. Eine der flachen Druckflächen wurde mit kleinen Vorsprüngen (Halbkugeln) längs des Umfangs versehen, um einen Mindestabstand zwischen den Platten sicherzustellen. Die gleiche Platte wurde auch mit einem Thermoelement (Ni, Cr—Ni) versehen, wobei der Messpunkt in der Mitte und teilweise in der Druckfläche angeordnet war. Die Temperatur wurde mit einem Drucker aufgezeichnet. Um eine gleichmässige Erwärmung zu erzielen, wurden die Pressplatten in einen Edelstahlkasten eingeschlossen (Wanddicke : 5 mm), der gerade gross genug war, um die beiden Pressplatten aufzunehmen. Dieses Verfahren ermöglichte es, die Temperaturunterschiede in einem Mittelbereich (65 × 65 mm) zwischen den zum Sintern verwendeten Platten kleiner als 10 °C zu halten. Faserflaum (Lieferant : Bilson AB Billesholm, Schweden) aus einer Alkaliglassorte mit 10 bis 15 % Alkali (Kronglas) mit einem Faserdurchmesser von 2 ± 1 Mikrometer wurde verwendet. Es wurde zwischen die Platten in den vorbestimmten Sinterbereich gebracht und mit 3 kPa zusammengepresst, d. h. mit dem durch das Gesamtgewicht der oberen Pressplatte und des oberen Teils des Stahlkastens erzeugten Druck. Der belastete Kasten wurde in einen Elektroofen gebracht, der automatisch auf 800 °C gehalten wurde. Wenn nach ca. 20 Minuten eine Temperatur von 685 ± 5 °C im Messpunkt erreicht wurde, wurde der Sintervorgang abgebrochen. Man liess den Kasten bei Raumtemperatur abkühlen, und bei ca. 300 °C wurde er geöffnet und die gesinterte Schicht herausgenommen. Aufgrund der Abstandsstücke in der Pressplatte war diese Schicht dann 775 ± 25 Mikrometer dick.

Die zwischen den Platten zum Erzielen der gewünschten Porosität erforderliche Glasfasermenge wurde unter Verwendung einer Glasdichte von 2,5 $g/cm^3$ berechnet. Es wurden zwei Porositätsgrade verwendet, wobei der eine der Sinterschicht einen mittleren Glas-Volumenanteil von ca. 40 % und der andere von ca. 56 % verlieh.

Die Sinterschichten wurden zerkleinert und mit einem Porzellanmörser und einer Mörserkeule zermahlen. Das Pulver wurde durch eine Reihe von Normsieben (DIN 4188) gesiebt, und die Teilmengen wurden nach der Maschenweite (in Mikrometer) des Siebes, an dem sie entnommen wurden, sowie der nächstgrösseren Maschenweite bezeichnet. Es wurde eine Teilmenge von « groben » (250/160), zwei « mittleren » (160/100, 100/71) und einer « feinen » (71/40) Grössensorte der Teilchen entnommen. Die Pulver wurden 24 Stunden lang mit Normalsalzsäure (1-M hydrochloric acid) behandelt und dann mit destilliertem Wasser und Aceton gewaschen.

Die zweckmässigste Teilchengrösseverteilung des Pulveranteils des Zahnfüllmaterials ist derart, dass die Teilchen vorzugsweise Durchmesser von weniger als 250 aber mehr als 10 Mikrometer aufweisen. Dies hängt jedoch in gewissem Grad von der Art des verwendeten porösen Glases ab, und in einigen Fällen können Teilchengrössen von weniger als 10 Mikrometer verwendet werden.

Eine Silan-Oberflächenbehandlung mit einem Gewichtsanteil von ca. 1/2 % des Pulvers wurde als in Aceton gelöstes Silan (Silan A 174, Union Carbide Co., Lot 803 080 174) beigefügt. Man liess das Pulver bei Raumtemperatur trocknen, wonach es 45 Minuten lang auf 110 °C erhitzt wurde. Wenn bei vorsichtigem Pressen das Pulver ein Tröpfchen destillierten Wassers nicht innerhalb von 5 Minuten eindringen liess, wurde die Silanbehandlung als befriedigend betrachtet.

Der Anteil der Ausbesserungszusammensetzung an anorganischen Teilchen sollte zwischen 40 und 90 % Volumenanteil, vorzugsweise zwischen 50 une 80 % Volumenanteil liegen. Bei einem erhöhten Volumenanteil wird der Glanz und die Härte der Glasoberfläche verbessert.

Folgende Beispiele sollen zeigen, wie die Erfindung in der Praxis angewandt wird.

Beispiele

Bis-GMA (Freeman Chemical Co., Port Washington, Wi., U.S.A., Lot 124543), ein Reaktionsprodukt von Bisphenol A und Glycydylmethacrylat, wie in der US-PS 3 006 112 beschrieben, wurde mit TEGDMA (Triäthylen-Glykol-Dimethacrylat) in dem in der folgenden Tabelle gegebenen Verhältnis verdünnt. Der Monomermasse wurde ein Photoinitiator (Benzoinmethyläther, 0,8 %) und ein Stabilisator (Hydrochinonmonomethyläther, 0,008 %) beigegeben. Kolloidales Siliziumdioxid (Aerosil® R 972), Degussa, Frankfurt, BRD) wurde in verschiedenen Mengen entsprechend den Angaben in der Tabelle zugesetzt, um den Anteil der anorganischen Bestandteile zu erhöhen und die Monomerviskosität abzustimmen.

Die in Tabelle 1 aufgeführten Pulver und Flüssigharze wurden mit einer Achat-Zahnarztspachtel auf einer Glasplatte gemischt. Zur « Sättigung » wurde Pulver bis zu dem Punkt zugefügt, an dem trockene Teilchen in der Mischung zu erscheinen begannen und damit das Fehlen einer zum Imprägnieren weiterer Teilchen erforderlichen Menge von überschüssigem Monomer anzeigten.

Die Pulver-Harz-Mischungen wurden in zylindrische Hohlräume (Durchmesser 4 mm, Tiefe 2 mm) in Acrylstäben (4 cm lang) mit quadratischem Querschnitt (1 × 1 cm) gefüllt. Zur Druckausübung wurde ein Amalgamverdichter mit einer flachen und kreisförmigen Verdichterspitze (Durchmesser 1,8 mm) verwendet. Die angewandte Kraft wurde durch Auflegen des Acrylstabes auf eine einfache Waage gemessen.

Die Verdichtung (Pressung) erfolgte rechtwinklig zum Boden des Hohlraumes. Sie begann in der Mitte, und die Verdichterspitze wurde dann schrittweise über die Oberfläche geführt, bis diese mit überlappenden Eindrücken bedeckt war. Dies wurde einmal wiederholt. Beim erstenmal wurde ein verhältnismässig niedriger Druck angewandt, um eine erste Verfestigung und eine ebene Oberfläche zu erzielen. Wenn der Höchstdruck (Tabelle 1) angewandt wurde, wurde er etwa 1 Sekunde lang konstant gehalten. Es wurden nacheinander jeweils Teilmengen eingeführt, die eine 1 bis 1,5 mm dicke Schicht verdichteten Materials ergaben. Jede Schicht wurde durch 60 bis 80 Sekunden lange Einwirkung von UV-Strahlung (Nuva-Lite®, L. D. Caulk Company, Kanada) ausgehärtet. Obwohl der verwendete Verfestigungsdruck in Tabelle 1 angegeben ist, kann dieser Druck je nach Teilchendichte schwanken. Z. B. können Teilchen mit höherer Dichte höhere Drücke über den gleichen Zeitraum, d. h. 1 Sekunde, wie oben beschrieben, erfordern.

(Siehe Tabelle 1 Seite 5 f.)

Die Hohlräume wurden mit einer Überschussfüllung von ca. 0,5 mm Dicke versehen, um die komplette Entfernung der Oberflächenschicht mit Sauerstoffinhibition bei der Oberflächenbearbeitung sicherzustellen. Einfüllen und Aushärten erfolgten bei Raumtemperatur (22 ± 1 °C). Der Überschuss wurde entfernt und das Muster wurde mit Karborundpapier Nr. 600 bündig zur Oberfläche des Acrylstabs geschliffen. Anschliessend erfolgte Polieren auf Polierleinen mit einer wässrigen Suspension von Korundpulver mit einer Teilchengrösse von 0,3 Mikrometer.

Es wurden Ätzmuster in der Mittelebene geschnitten und die freigelegte Querschnittsfläche wurde geschliffen. Dann wurde das Muster mit kalthärtendem Acrylharz befestigt und wie zuvor geschliffen. Die Oberfläche, die einen Querschnitt des Musters zeigte, wurde mit Fluorwasserstoffsäure (40 %) 1/2 Stunde lang geätzt und in destilliertem Wasser gespült.

Auf der polierten Fläche des Musters wurde die Härte mit einem Vickers-Härteprüfer (Typ Z3, 2A, Zwick & Co., Einsingen, üb. Ulm/Donau, BRD) nach DIN 50133 mit einer Belastung von 10 daN (10 kp) gemessen. Vor dem Prüfen wurden die Muster eine Woche lang gelagert (22 ± 1 °C und 50 ± 5 % relative Feuchtigkeit).

Die geätzten Flächen wurden bei schräg einfallendem Licht mikroskopisch untersucht (× 40). Mit diesem Verfahren zeigte sich die Grenzfläche Harz/Teilchen klar definiert.

Zum Prüfen der Polierbarkeit wurden Musterflächen mit Aluminiumoxidscheiben (Sof-Lex®, Korngrösse mittelfein, fein und hochfein. 3M Co., St. Paul, Minnesota, U.S.A.) geschliffen und dann mit einer Weissgummischeibe (Identoflex®, Typ 1008, extrafein, Identoflex AG, Buchs, Schweiz) poliert, beides bei niedriger Drehzahl.

Für alle verwendeten Ansätze ergab das Verfahren eine Masse, die eine Berührung zwischen den einzelnen Teilchen 1 aufwies. In den meisten Fällen waren sie in sehr guten Kontakt gebracht, so dass keine freien Zonen zwischen den Teilchen sichtbar waren. Eine Veränderung der Härtezahl war vorwiegend die Folge von Schwankungen des anorganischen Anteils.

Bei allen Ansätzen wies das verdichtete Material eine feste Oberfläche auf, die mit der Spitze eines Amalgamverdichters geformt werden konnte. Alle Flächen, die bei Polieren mit Gummischeiben erzeugt wurden, zeigten eine glänzende Oberflächenglätte, die mit steigender Härtezahl eine zunehmende Tendenz aufwies.

Die Vickers-Härtezahl des Polymers, das den verschiedenen Flüssigharzmassen entsprach, schwankte nur in geringem und unbedeutendem Ausmass, wie eine Vorabstudie zeigte. Die Härtezahl spiegelt daher haupsächlich die Konzentration der porösen Teile und die Dichte der einzelnen Teilchen 1 wider.

Die Teilchenkonzentration, wie sie sich sowohl in der Härtezahl als im mikroskopsichen Aussehen widerspiegelte, war für die verschiedenen verwendeten Flüssigharze 2 (Masse 2-4) ungefähr gleich. Es ergab sich auch keine Änderung, wenn eine Teilmenge von « mittelgrossen » Teilchen mit einbezogen wurde (Masse 5). Dagegen ergab sich eine wesentliche Härtezunahme, wenn « Teilchen von hoher Dichte » verwendet wurden (Masse 6).

Bei allen Flüssigharzen 2 war die Verdünnermenge klein im Verhältnis zu der allgemein verwendeten Menge. Das Verdichtungsverfahren konnte selbst dann durchgeführt werden, wenn die Viskosität durch Zufügen von kolloidalem Siliziumdioxid weiter erhöht wurde. Daraus ergibt sich die Wahl zwischen einer oder beiden von zwei Möglichkeiten, von denen die erste in der Verwendung eines Monomers mit höherem durchschnittlichem Molekulargewicht als üblicherweise verwendet, und die zweite in der Erhöhung der Konzentration des anorganischen Bestandteils durch Zufügen einer Teilmenge von Teilchen besteht, die fein genug sind, um das poröse Fasernetz zu durchdringen. Zum letztgenannten Zweck können Teilchen mit sowohl kolloidalen als auch nichtkolloidalen Grössen und vorzugsweise mit hoher Strahlungsundurchlässigkeit verwendet werden.

Reduzierter Schrumpf bei der Polymerisation und eine geringere Tendenz zu Farbverschiebungen kann z. B. für Monomere auf Bis-GMA-Basis erzielt werden, die ein höheres mittleres Molekulargewicht (und dadurch auch eine höhere Viskosität) als die allgemein verwendeten herkömmlichen Monomere aufweisen. Eine solche Zunahme des Molekulargewichtes könnte durch eine Änderung der Menge und der Molekülgrösse des Verdünnungsmittels und/oder durch Zufügung von noch grösseren Molekülen als Bis-GMA erreicht werden. Ein geringerer Schrumpf während der Polymerisation ergibt einen geringeren Aufbau von inneren Spannungen und eine verringerte Hohlraumbildung. Durch eine Erhöhung des mittleren Molekulargewichts wird auch die mechanische Festigkeit verbessert und die Tendenz zur Sauerstoffinhibition bei der Polymerisation wird verringert.

4

## Tabelle 1

Aussehen nach Ätzung und Eigenschaften der Verbundmassenmuster, die poröse Teilchen mit verschiedener Grösse, Verteilung und Dichte enthalten.

| Mischungsansatz | | | | | | | Eigenschaften der Muster | | | |
|---|---|---|---|---|---|---|---|---|---|---|
| Verbund-masse Nr. | Poröse Teilchen Typ | Teilmengen | TEGDMA in Bis-GMA % Gew. | Kolloidales Siliziumdioxid in Flüssigharz (% Gew.) | Mischungs-eigenschaften | Einsetz-verfahren | Mikroskopisches Aussehen nach dem Ätzen | Härte ($H_{V10}$) Mittelwert[x] | S.D.$_{.2}$ (kp/mm²) |
| 1 | Geringe Dichte | Grob (250/160,70%) Mittel (160/100,20%) (100/71,10%) | 8 | 11 | Mit Pulver gesättigt | $\cong$ 15 MPa Verdich-tungs-druck | Teilchen in Berührung | 209 | 9,6 | |
| 2 | do. | Grob (250/160,70%) Fein (71/40,30%) | 8 | 11 | do. | do. | Keine Leer-zonen zwischen Teilchen sichtbar | 232 | 7,6 | |
| 3 | do. | do. | 8 | 19 | do. | do. | do. | 244 | 15,1 | |
| 4 | do. | do. | 20 | 19 | do. | do. | do. | 247 | 4,6 | |
| 5 | do. | Grob (250/160,52%) Mittel (160/100, 26%) Fein (71/40,22%) | 8 | 11 | do. | do. | do. | 249 | 16,7 | |
| 6 | Hohe Dichte | do. | 8 | 19 | do. | do. | do. | 293 | 9.0 | |

[x]) Alle Werte aus 6 Messungem gemittelt.

0 048 681

Es hat sich auch durch Versuche gezeigt, dass es möglich ist, Pulver-Monomer-Mischungen, die einen wesentlichen Monomerüberschuss über die zum Füllen der porösen Teilchen 1 erforderliche Menge hinaus enthalten, auf die gewünschte Struktur zu verdichten, vorausgesetzt, dass es die Viskosität des Harzes gestattet, mindestens einen Teil des überschüssigen Harzes im Zusammenhang mit der Verdichtung durch das Netz zu pressen. Sowohl in diesen Fällen als auch dann, wenn kein überschüssiges Monomer vorhanden ist, kann die Verdichtung dergestalt ausgeführt werden, dass der grösste Teil der Grenzflächen der porösen anorganischen Teilchen 1 miteinander in Berührung gebracht wird, möglicherweise in Verbindung mit weitgehender Quetschung der Oberflächenschicht der Teilchen. Die Teilchenquetschung kann so weit gehen, dass Teilchen sich derart aneinander anpassen, dass praktisch keine Leerräume zwischen den Teilchen übrigbleiben.

Es versteht sich, dass die Teilchen nicht nur durch mechanische Verzahnung sondern auch durch die Tatsache zusammengehalten werden, dass zwischen dem Harz und dem Glas aufgrund einer Silanbehandlung (organisch-funktioneller Adhäsionsaktivator) eine Haftfähigkeit besteht. Eine derartige Behandlung ist nach dem Stand der Technik bekannt, war jedoch bisher nicht in der Lage, bei normalen Arten von Verbundmassen ausreichend starke Bindungen zu ergeben. Die Erfindung verwendet nicht nur diese Klebeverbindung, sondern eine sich aus dem Verfahren gemäss der Erfindung ergebende mechanische Verzahnung. Bei der bevorzugten Ausführungsform der Erfindung kann der Durchmesser der Kanalporosität schwanken, solange als die Teilchen unter mehr oder weniger starker Verformung eng gepackt sind, während gleichzeitig der Druck im Harz durch plastisches Fliessen des Überschussharzes durch die Poren des anorganischen Netzes ausgeglichen wird.

Andere Verfahren als die im vorliegenden Beispiel zur Einleitung der Polymerisationsreaktion verwendete Photoinitiation können ebenfalls verwendet werden. Ein Beispiel für ein solches Verfahren ist die Warmaushärtung, bei der die für die Polymerisation erforderlichen freien Radikale beim Erhitzen eines Monomers entstehen, das z. B. Peroxid enthält. Bei einem anderen Verfahren wird die Masse zunächst in zwei Teilmengen unterteilt, wobei die erste Teilmenge zusätzlich zu einem in Wärme aushärtenden Kunstharz und zu anorganischen Bestandteilen einen Katalysator für das in Wärme aushartende Kunstharz, und die zweite Teilmenge ein Aktivierungsmittel für den Katalysator enthält. Die Polymerisation beginnt dann beim Mischen der beiden Teilmengen. Die Auswahl des speziellen Katalysators und des Aktivierungsmittels sowie deren Mengen liegt im Rahmen des Standes der Technik und hängt von der speziellen verwendeten Harzmenge ab.

Bei dem Verfahren gemäss der Erfindung ist es auch möglich, poröse anorganische Teilchen 1 zu verwenden, die nur in den äusseren Bereichen aushärtbares Harz 2 und in den Mittelbereichen gehärtetes Harz 2 enthalten.

Zur Erhöhung der Haftfähigkeit zwischen den anorganischen Bestandteilen und dem Harz können die ersteren mit einem geeigneten organischfunktionellen Silan-Haftvermittler vorbehandelt werden, wie weiter oben beschrieben. Es kann jedoch mindestens ein Teil des Silans auch dem Harz beigegeben werden.

Die porösen anorganischen Teilchen 1 können mit thermoplastischem Harz 2 imprägniert werden, und beim Erhitzen kann die Masse dergestalt zusammengepresst werden, dass die porösen anorganischen Teilchen zusammengeklebt werden.

Die anorganischen Teilchen im aushärtbaren Harz 2 umfassen Keramikteilchen, vorzugsweise aus Aluminiumoxid oder Siliziumoxid ($SiO_2$).

Das aushärtbare Harz 2 kann im wesentlichen mindestens ein Monomer und/oder Polymer enthalten, das aus einer Gruppe gewählt ist, die Acrylharz, Vinylchlorid, Zelluloseazetat, Styrol und Acrylsäurenitril-Copolymerisat umfasst. Das aushärtbare Harz 2 kann auch mindestens ein Acryl-Monomer und/oder Polymer enthalten, das aus der Gruppe gewählt ist, die Äthyl-Methacrylsäureester, Äthylacrylat und Methyl-Methacrylsäureester umfasst. Das aushärtbare Harz 2 kann im wesentlichen ein Copolymerisat eines Acryl-Monomers und eines anderen copolymerisierbaren Monomers sein. Das copolymerisierbare Monomer ist aus der Gruppe gewählt, die aus Styrol, Butadien, Äthylen und Acrylsäure besteht.

Das aushärtbare Harz 2 enthält im wesentlichen Bis-GMA oder irgendein anderes Derivat von Bisphenol A, und die Aushärtung kann durch Photoinitiation einer Polymerisationsreaktion erreicht werden.

Das Verfahren gemäss der Erfindung wird vorzugsweise zur Durchführung von Zahnausbesserungen wie Füllungen, Verkleidungen, künstlichen Zähnen und dergleichen verwendet. Zur Erzielung einer Zahnausbesserung mit verbesserten mechanischen, physikalischen und ästhetischen Eigenschaften sowie mit verbesserten Eigenschaften bezüglich der klinischen Handhabung zeichnet sich das Verfahren dadurch aus, dass ein zahnärztliches Verbundharz im wesentlichen aus porösen anorganischen Teilchen besteht, die vollständig oder teilweise mit einem mindestens teilweise polymerisierbaren Monomer und/oder Polymer imprägniert werden, und dass die Teilchen verdichtet oder auf andere Weise dergestalt aufgetragen werden, dass sie sich gegenseitig berühren und bei der Polymerisation zusammenkleben, um eine Harzstruktur mit einer angrenzenden anorganischen Phase zu bilden.

Es hat sich im Laboratorium und bei klinischen Versuchen erwiesen, dass es mit dem beschriebenen Verfahren möglich ist, zahnärztliche Ausbesserungen durchzuführen, deren mechanische, physikalische und ästhetische Eigenschaften weitgehend dem natürlichen Zahngefüge entsprechen. Ausserdem wird ein nur unbedeutender Schrumpf bei der Polymerisation und eine hervorragende Polierfähigkeit erzielt.

Diese Eigenschaften wurden bei den Verfahren und Massen nach dem Stand der Technik nicht gleichzeitig erzielt.

Das Verfahren der mechanischen Verfestigung und Verdichtung eines Materials in einer Zahnaushöhlung ist den Zahnärzten im Zusammenhang mit Zahnamalgam bekannt. Es weist bekannte Vorteile insofern auf, als es eine enge Anpassung des Füllmaterials an die Wände der Aushöhlung gestattet und es ermöglicht, einen festen Kontakt zwischen dem ausgebesserten Zahn und dem benachbarten Zahn herzustellen. Ferner ermöglicht es dieses Verfahren, der Ausbesserung ihre endgültige anatomische Form vor dem Aushärten zu geben. Dabei erübrigt sich zeitraubende und schwierige Nachbearbeitung mit rotierenden Instrumenten. Diese Vorteile werden auch mit dem Verfahren gemäss der Erfindung erzielt, was bisher mit zahnähnlichem Ausbesserungsmaterial nicht erreichbar war.

Die mechanische Verzahnung der anorganischen Bestandteile (insbesondere mit der als Beispiel beschriebenen Art von porösen Teilchen), die während eines Verdichtungsvorganges erreicht werden kann, ergibt eine Masse, die selbst vor dem Aushärten eine feste Konsistenz aufweist, was eine äusserst wünschenswerte Eigenschaft darstellt, da die klinische Handhabung dadurch erleichtert wird. Das Einführen in die Aushöhlung und das Verdichten wird erleichtert, wenn das material in geeigneten Formen vorverdichtet wird, um kleine Klümpchen oder Tabletten zu formen. Demzufolge können dem Material verschiedene Grade von Bindekraft verliehen werden, mit oder ohne Oberflächeneinbeulung der porösen Teilchen.

Die bevorzugte Ausführungsform ist besonders vorteilhaft wegen der hervorragenden Fähigkeit der Teilchen, sich gegenseitig zu verzahnen, zusammenzuhängen und eng gepackt zu werden, und weiterhin wegen der sich ergebenden Porenstruktur, in der sich der Druck im organischen Flüssigharz leicht ausgleicht. Die Verdichtung kann daher klinish durch Verwendung von Drücken von weniger als 20 MPa durchgeführt werden, was für das klinische Verfahren, die Verarbeitbarkeit und die Schonung des Patienten wichtig ist. Die Drücke werden in MPa gemessen, wobei $1 \text{ kP/mm}^2 = 9{,}806\,65 \text{ MPa}$.

Eine elektronenmikroskopische Analyse des gesinterten Produkts nach dem beschriebenen Beispiel, wobei der Faserdurchmesser weniger als 4 Mikrometer betrug, zeigt, dass die Fasern miteinander verschmolzen sind und ein Glasskelett bilden, das von einem Mikroporen-System durchzogen ist. Dieses Glasskelett kann als Retentionsmittel verwendet werden und weist eine in Bezug auf Festigkeit, Steifheit und Haftfähigkeit günstige Struktur auf. Die Hohlräume des Skeletts oder Netzes sind durchgehend angeordnet, was das Material zur Tiefimprägnation mit Harzen befähigt, erforderlichenfalls unter Anwendung eines verringerten Drucks, d. h. durch Anlegen eines Vakuums. Es ist vorzuziehen, die Tiefimprägnierung mit grösseren Einheiten der Netzstruktur durchzuführen. Die imprägnierte grössere Netzstruktur wird dann ausgehärtet und nach Umformung zu einem teilchenförmigen Füllmaterial gemahlen. Das Imprägnierungsmittel (Harz) des Füllmaterials (d. h. des zum Imprägnieren des Glasskeletts verwendeten Harzes) braucht nicht das gleiche zu sein wie das zum Herstellen des Massegefüges verwendete Mittel.

Neben der Verwendung als teilchenförmiges Füllmaterial kann das anorganische Netz in grossprofiligen Teilen zum Füllen, Versteifen und Verfestigen von zahnärztlichem Ausbesserungs- und Aufbaumaterial sowie auch als Material für Implantate verwendet werden. Die bemerkenswerten Eigenschaften, die mit dem im Beispiel genannten Material mit einem Faserdurchmesser von weniger als 4 Mikrometer und insbesondere einem mittleren Faserdurchmesser von 2 Mikrometer, beim Einsetzen in hartes Knochengewebe erzielbar sind, wurden von Ehrnford et al. in einer Veröffentlichung mit dem Titel « Ausbildung von Knochengewebe in einem in Extraktionssockeln bei Ratten eingepflanzten gesinterten Mikroporen-Glasfasernetz », Scand. J. Dent. Res. 1980 : 88 : 130-133, beschrieben. Hier wurde Knochengewebe gezeigt, wie es in die äusserst feinen Maschen von Zellgrösse einwuchs, wodurch es erstmalig möglich wird, Verbundmaterial an Knochengewebe zu verankern.

**Patentansprüche**

1. Verfahren zur Herstellung einer Verbundmasse aus organischem Harz und anorganischen porösen Teilchen, vorzugsweise zur Verwendung als zahnärztliches Ausbesserungsmaterial, dadurch gekennzeichnet, dass poröse anorganische Teilchen mit mindestens teilweise aushärtbarem Harzmaterial imprägniert, zusammengepresst oder auf andere Weise einem Druck dergestalt unterworfen werden, dass die Teilchen sich gegenseitig berühren und der Druck im Harz durch plastisches Fliessen des überschüssigen Harzes durch die Poren der anorganischen Teilchen ausgeglichen wird, und dass die Teilchen durch Aushärten mindestens eines Teils des aushärtbaren Harzmaterials zusammengeklebt werden und dadurch eine Harzstruktur mit einer angrenzenden, mit Harz imprägnierten anorganischen Phase bilden, wobei die porösen anorganischen Teilchen unter Ausschluß aller anderen porösen Füllstoffe ein starres dreidimensionales Netz von anorganischen Fasern enthalten, die zusammengeschweisst sind durch Erhitzen auf eine ausreichende Temperatur, um das Schmelzen und vollständige Zusammenschweissen im wesentlichen aller Einzelfasern an ihren Berührungspunkten zu einem Netz zu bewirken, in dem die Porosität durchgehend durch den ganzen Teil an den Berührungspunkten verläuft, an denen die Fasern gegeneinander anliegen.

2. Verfahren nach Anspruch 1, dadurch gekennzeichnet, dass die porösen anorganischen Teilchen dergestalt miteinander in Berührung gebracht werden, dass der grösste Teil ihrer Grenzfläche gegeneinander anliegt.

3. Verfahren nach Anspruch 2, dadurch gekennzeichnet, dass die porösen anorganischen Teilchen dergestalt miteinander in Berührung gebracht werden, dass sie durch Eindrücken der Oberflächen einen sehr guten Kontakt miteinander eingehen.

4. Verfahren nach Anspruch 3, dadurch gekennzeichnet, dass die Teilchen derart eingedrückt werden, dass sie sich weitgehend aneinander anpassen, so dass praktisch keine Zwischenräume zwischen den Teilchen freibleiben.

5. Verfahren nach Anspruch 1, dadurch gekennzeichnet, dass die porösen anorganischen Teilchen dergestalt mit Harzmaterial imprägniert werden, dass sich ein Überschuss an Harzmaterial bildet.

6. Verfahren nach Anspruch 1, dadurch gekennzeichnet, dass die porösen anorganischen Teilchen einen Durchmesser von weniger als 500 Mikrometer aufweisen.

7. Verfahren nach Anspruch 6, dadurch gekennzeichnet, dass die porösen anorganischen Teilchen einen Durchmesser von weniger als 300 Mikrometer aufweisen.

8. Verfahren nach Anspruch 7, dadurch gekennzeichnet, dass ein Gewichtsanteil von 10-90 % der porösen anorganischen Teilchen einen Durchmesser von mindestens 10 Mikrometer, aber weniger als 100 Mikrometer aufweist.

9. Verfahren nach Anspruch 8, dadurch gekennzeichnet, dass ein Gewichtsanteil von 10-40 % der porösen anorganischen Teilchen einen Durchmesser von mindestens 10 Mikrometer, aber weniger als 100 Mikrometer aufweist.

10. Verfahren nach Anspruch 1, dadurch gekennzeichnet, dass die Fasern aus einem Keramikmaterial hergestellt werden.

11. Verfahren nach Anspruch 10, dadurch gekennzeichnet, dass das Keramikmaterial aus Glas besteht.

12. Verfahren nach Anspruch 11, dadurch gekennzeichnet, dass das Glas gemäss einer Bestimmung durch Röntgenstrahlen eine hohe Dichte aufweist.

13. Verfahren nach Anspruch 12, dadurch gekennzeichnet, dass die Fasern im Netz einen Durchmesser von weniger als 100 Mikrometer aufweisen.

14. Verfahren nach Anspruch 13, dadurch gekennzeichnet, dass die Fasern einen Durchmesser von weniger als 10 Mikrometer aufweisen.

15. Verfahren nach Anspruch 14, dadurch gekennzeichnet, dass die Fasern einen Durchmesser von weniger als 4 Mikrometer aufweisen.

16. Verfahren nach Anspruch 15, dadurch gekennzeichnet, dass die Fasern einen Durchmesser von 1-3 Mikrometer aufweisen.

17. Verfahren nach Anspruch 1, dadurch gekennzeichnet, dass das aushärtbare Harzmaterial nichtporöse anorganische Teilchen enthält.

18. Verfahren nach Anspruch 17, dadurch gekennzeichnet, dass die anorganischen Teilchen im aushärtbaren Harzmaterial Keramikteilchen enthalten.

19. Verfahren nach Anspruch 18, dadurch gekennzeichnet, dass die Keramikteilchen aus Aluminiumoxid oder Siliziumdioxid ($SiO_2$) bestehen.

20. Verfahren nach Anspruch 19, dadurch gekennzeichnet, dass die Keramikteilchen Glasteilchen sind.

21. Verfahren nach Anspruch 18, dadurch gekennzeichnet, dass die anorganischen Teilchen im aushärtbaren Harzmaterial eine hohe Strahlenundurchlässigkeit für Röntgenstrahlen aufweisen.

22. Verfahren nach Anspruch 18, dadurch gekennzeichnet, dass die anorganischen Teilchen im aushärtbaren Harzmaterial eine Grösse unter 2 Mikrometer aufweisen.

23. Verfahren nach Anspruch 22, dadurch gekennzeichnet, dass die anorganischen Teilchen im aushärtbaren Harzmaterial innerhalb eines Grössenbereiches von 0,005 bis 0,4 Mikrometer liegen.

24. Verfahren nach Anspruch 22, dadurch gekennzeichnet, dass die anorganischen Teilchen im aushärtbaren Harzmaterial 10 bis 90 % des Gesamtgewichtes der Teilchen/Harz-Mischung ausmachen.

25. Verfahren nach Anspruch 1, dadurch gekennzeichnet, dass die anorganischen porösen Teilchen mit einem anorganisch-funktionellen adhäsionsaktivierenden Silan-Haftvermittler vorbehandelt wurden, wobei ein chemischer Adhäsionsmechanismus und ein Klebemechanismus mit physikalischer Durchdringung und Verzahnung zur verbesserten Geschlossenheit und Dauerhaftigkeit der porösen anorganischen Teilchen in Verbindung mit einem organischen Harz beiträgt.

26. Verfahren nach Anspruch 25, dadurch gekennzeichnet, dass mindestens ein Teil des Silan-Haftvermittlers in einem Gewichtsteil von 0,05 bis 10 % in Bezug auf das Gewicht der Gesamtmasse beigemengt ist.

27. Verfahren nach Anspruch 1, dadurch gekennzeichnet, dass die porösen anorganischen Teilchen einen Volumenanteil von 30 bis 80 % der anorganischen Substanz enthalten.

28. Verfahren nach Anspruch 17, dadurch gekennzeichnet, dass die Grösse der porösen anorganischen Teilchen kleiner als 300 Mikrometer ist.

29. Verfahren nach Anspruch 1, dadurch gekennzeichnet, dass die porösen anorganischen Teilchen in ihrem äusseren Bereich aushärtbares Harzmaterial und im Mittelbereich gehärtetes Harzmaterial

**0 048 681**

enthalten.

30. Verfahren nach Anspruch 1, dadurch gekennzeichnet, dass die porösen anorganischen Teilchen mit einem thermoplastischen Harz imprägniert sind, und dass die Verdichtung der Masse während des Erhitzens dergestalt stattfindet, dass die porösen anorganischen Teilchen zusammengeklebt werden.

31. Verfahren nach Anspruch 1 oder 17, dadurch gekennzeichnet, dass das aushärtbare Harzmaterial im wesentlichen mindestens 1 Monomer und/oder Polymer enthält, das aus der Gruppe gewählt ist, die aus Acrylharz, Vinylchlorid, Zelluloseazetat, Styrol, und Acrylsäurenitril-Copolymerisat besteht.

32. Verfahren nach Anspruch 31, dadurch gekennzeichnet, dass das aushärtbare Harz mindestens 1 Acryl-Monomer und/oder Polymer ist, das aus der Gruppe gewählt ist, die aus Äthyl-Methacrylsäureester, Äthylacrylat und Methyl-Methacrylsäureester besteht.

33. Verfahren nach Anspruch 31, dadurch gekennzeichnet, dass das aushärtbare Harzmaterial im wesentlichen ein Copolymerisat eines Acryl-Monomers und eines anderen copolymerisierbaren Monomers ist.

34. Verfahren nach Anspruch 31, dadurch gekennzeichnet, dass das aushärtbare Harz ein Copolymerisat eines Acryl-Monomers und eines anderen copolymerisierbaren Monomers ist, das aus der Gruppe gewählt ist, die aus Styrol, Butadien, Äthylen und Acrylsäure besteht.

35. Verfahren nach Anspruch 31, dadurch gekennzeichnet, dass das aushärtbare Harzmaterial im wesentlichen Bis-GMA oder ein beliebiges sonstiges Derivat von Bisphenol A enthält.

36. Verfahren nach Anspruch 1, dadurch gekennzeichnet, dass das Aushärten des Harzes durch Photoinitiation einer Polymerisationsreaktion erzielt wird.

37. Verfahren nach Anspruch 1, dadurch gekennzeichnet, dass ein Verbundharz, das im wesentlichen aus porösen anorganischen Teilchen besteht, die vollständig oder teilweise mit einem mindestens zum Teil polymerisierbaren Monomer und/oder Polymer inprägniert sind, und die verdichtet oder auf andere Weise dergestalt aufgetragen werden, dass die Teilchen sich gegenseitig berühren und bei der Polymerisation zusammengeklebt werden, um eine Harzstruktur mit einer angrenzenden anorganischen Phase zu bilden.

**Claims**

1. A method of producing a composite of organic resin and inorganic porous particles, perferably for use as a dental restoration material, which comprises impregnating porous inorganic particles with an at least partially hardenable resin material, compressing or otherwise applying pressure in such a way that the particles contact each other and the pressure in the resin is equalized by viscous flow of excess resin through the pores of the inorganic particles, bonding together by hardening of at least a portion of the hardenable resin material thereby forming a resin structure including a contiguous resin impregnated inorganic phase, whereby the porous inorganic particles excluding any other porous fillers comprise a rigid three-dimensional network of inorganic fibres fused together by heating at a temperature sufficient to cause melting and complete fusing together of substantially all individual fibres at their points of contact into a network where the porosity is continuous throughout the entire element in those contact points at which the fibres engage each other.

2. The method as claimed in claim 1, wherein the porous inorganic particles are brought in contact with each other such that the major part of their interfaces contact each other.

3. The method as claimed in claim 2, characterized in that the porous inorganic particles are brought in contact with each other such that they obtain intimate contact by surface crushing.

4. The method as claimed in claim 3, wherein the particle crushing is such that the particles conform to each other to an extent that practically no interparticle spaces are left.

5. The method as claimed in claim 1, wherein the porous inorganic particles are impregnated with resin material such that excess of resin material is formed.

6. The method as claimed in claim 1, wherein the porous inorganic particles have a diameter of less than 500 microns.

7. The method as claimed in claim 6, wherein the porous inorganic particles have a diameter of less than 300 microns.

8. The method as claimed in claim 7, wherein 10-90 % by weight of the porous inorganic particles have a diameter of at least 10 microns but less than 100 $\mu$m.

9. The method as claimed in claim 8, wherein 10-40 % by weight of the porous inorganic particles have a diameter of at least 10 $\mu$m but less than 100 $\mu$m.

10. The method as claimed in claim 1, wherein the fibres are made of a ceramic material.

11. The method of claim 10, wherein the ceramic material is glass.

12. The method as claimed in claim 11, wherein the glass has a high density as determined by X-rays.

13. The method as claimed in claim 12, wherein the fibres in the networks have a diameter of less than 100 $\mu$m.

14. The method as claimed in claim 13, wherein the fibres have a diameter of less than 10 $\mu$m.

15. The method as claimed in claim 14, wherein the fibres have a diameter of less than 4 $\mu$m.

16. The method of claim 15, wherein the fibres have a diameter of from 1-3 $\mu$m.

17. The method as claimed in claim 1, wherein the hardenable resin material contains nonporous inorganic particles.

18. The method as claimed in claim 17, wherein the inorganic particles in the hardenable resin material comprise ceramic particles.

19. The method as claimed in claim 18, wherein the ceramic particles are of aluminiumoxide or siliconedioxide $(SiO_2)$.

20. The method as claimed in claim 19, wherein the ceramic particles are glass particles.

21. The method as claimed in claim 18, wherein the inorganic particles in the hardenable resin material have a high radiopacity to X-rays.

22. The method as claimed in claim 18, wherein the inorganic particles in the hardenable resin material have a size below 2.

23. The method as claimed in claim 22, wherein the inorganic particles in the hardenable resin material are within the size range of 0.005 to 0.4 $\mu$m.

24. The method as claimed in claim 22, wherein the inorganic particles of the hardenable resin comprise 10 to 90 percent of the total weight of the particle resin mixture.

25. The method as claimed in claim 1, wherein the inorganic porous particles have been pretreated with an organofunctional silane adhesion promoting coupling agent whereby a chemical adhesion mechanism and a bonding mechanism involving physical penetration and interlocking contribute to the improved integrity and durability of the porous inorganic particles in combination with an inorganic resin.

26. The method as claimed in claim 25, wherein at least a portion of the silane bonding agent is incorporated in the resin in an amount of 0.05 to 10 % by weight based on the weight of the total composition.

27. The method as claimed in claim 1, wherein the porous inorganic particles contain 30-80 % by volume of inorganic substance.

28. The method as claimed in claim 17, wherein the size of the porous inorganic particles is less than 300 $\mu$m.

29. The method as claimed in claim 1, wherein the porous inorganic particles in their outer portions contain hardenable resin material and in their central portion hardened resin material.

30. The method according to claim 1, wherein the porous inorganic particle are impregnated with a thermoplastic resin and that the compression of the mass occurs during heating is such that the porous inorganic particles are bonded together.

31. The method as claimed in claim 1 or 17 wherein the hardenable resin material comprises substantially at least one monomer and/or polymer selected from the group consisting of acrylic resin, vinyl chloride, cellulose acetate, styrene and acrylonitrile copolymer.

32. The method as claimed in claim 31, wherein the hardenable resin is at least one acrylic monomer and/or polymer selected from the group consisting of ethyl methacrylate, ethyl acrylate, and methyl methacrylate.

33. The method as claimed in claim 31, wherein the hardenable resin material is substantially a copolymer of an acrylic monomer and another copolymerizable monomer.

34. The method as claimed in claim 31, wherein the hardenable resin is copolymer of an acrylic monomer and another copolymerizable monomer selected from the group consisting of styrene, butadiene, ethylene and acrylic acid.

35. The method as claimed in claim 31, wherein the hardenable resin material comprises substantially Bis-GMA or any other derivative of Bisphenol A.

36. The method as claimed in claim 1, wherein the hardening of the resin, is achieved through photoinitiation of a polymerization reaction.

37. The method as claimed in claim 1, wherein a composite resin consisting essentially of porous inorganic particles which are completely or partially impregnated with an at least partially polymerizable monomer and/or polymer and which particles are condensed or otherwise applied in such a way that the particles contact each other and on polymerization are bonded together to form a resin structure including a contiguous inorganic phase.

**Revendications**

1. Procédé de préparation d'une masse composite de résine organique et de particules minérales poreuses destinée de préférence pour être employée comme matériau pour travaux de prothèses dentaires, procédé caractérisé en ce que les particules minérales poreuses sont imprégnées par une résine au moins partiellement durcissable, compressées ou soumises d'une autre manière à une pression, que ces particules sont en contact mutuel et que la pression dans la résine s'équilibre par le fluage plastique de la résine en excès dans les pores des particules minérales, et enfin que les particules se collent ensemble par durcissement d'au moins une partie de la résine durcissable et qu'ainsi elles forment une structure de résine avec une phase minérale contiguë imprégnée par une résine, les particules minérales poreuses excepte tous autres matériaux de remplissage poreux contiennent un réseau tridimensionnel rigide de fibres minérales qui sont soudées ensemble, par chauffage, à une température appropriée pour fondre et souder ensemble essentiellement toutes les fibres individuelles en leurs points de contact, en un réseau dans lequel la porosité s'étend d'une façon continue dans toute la partie où se trouvent des points de contact où les fibres s'appliquent les unes contre les autres.

2. Procédé selon la revendication 1, caractérisé en ce que les particules minérales poreuses sont amenées au contact les unes avec les autres, de façon que la plus grande partie de leurs surfaces limitrophes s'appliquent les unes contre les autres.

3. Procédé selon la revendication 2, caractérisé en ce que les particules minérales poreuses sont amenées en contact les unes avec les autres de façon que par la pénétration sous pression des surfaces, il s'établisse un très bon contact réciproque.

4. Procédé selon la revendication 3, caractérisé en ce que les particules sont compressées de façon telle qu'elles s'ajustent dans une grande mesure les unes dans les autres, de sorte qu'il ne reste pratiquement aucun espace libre entre les particules.

5. Procédé selon la revendication 1, caractérisé en ce que les particules minérales poreuses sont imprégnées de résine, de telle façon qu'il se forme un excès en matière résineuse.

6. Procédé selon la revendication 1, caractérisé en ce que les particules minérales poreuses présentent un diamètre inférieur à 500 μm.

7. Procédé selon la revendication 6, caractérisé en ce que les particules minérales poreuses présentent un diamètre inférieur à 300 μm.

8. Procédé selon la revendication 7, caractérisé en ce qu'une proportion en poids de 10 à 90 % des particules minérales poreuses présente un diamètre d'au moins 10 μm mais inférieur à 100 μm.

9. Procédé selon la revendication 8, caractérisé en ce qu'une proportion en poids de 10-40 % des particules minérales poreuses présente un diamètre d'au moins 10 μm mais inférieur à 100 μm.

10. Procédé selon la revendication 1, caractérisé en ce que les fibres sont fabriquées à partir d'un matériau céramique.

11. Procédé selon la revendication 10, caractérisé en ce que le matériau céramique est constitué par du verre.

12. Procédé selon la revendication 11, caractérisé en ce que le verre présente une densité élevée, déterminée au moyen de rayons X.

13. Procédé selon la revendication 12, caractérisé en ce que les fibres du réseau présentent un diamètre inférieur à 100 μm.

14. Procédé selon la revendication 13, caractérisé en ce que les fibres présentent un diamètre inférieur à 10 μm.

15. Procédé selon la revendication 14, caractérisé en ce que les fibres présentent un diamètre inférieur à 4 μm.

16. Procédé selon la revendication 15, caractérisé en ce que les fibres présentent un diamètre de 1 à 3 μm.

17. Procédé selon la revendication 1, caractérisé en ce que la résine durcissable contient des particules minérales non poreuses.

18. Procédé selon la revendication 17, caractérisé en ce que les particules minérales dans la résine durcissable contiennent des particules céramiques.

19. Procédé selon la revendication 18, caractérisé en ce que les particules céramiques sont constituées d'oxyde d'aluminium ou de dioxyde de silicium ($SiO_2$).

20. Procédé selon la revendication 19, caractérisé en ce que les particules céramiques sont des particules de verre.

21. Procédé selon la revendication 18, caractérisé en ce que les particules minérales renfermées dans la résine durcissable présentent une grande imperméabilité aux rayons X.

22. Procédé selon la revendication 18, caractérisé en ce que les particules minérales renfermées dans la résine durcissable présentent une dimension inférieure à 2 μm.

23. Procédé selon la revendication 22, caractérisé en ce que les particules minérales renfermées dans la résine durcissable présentent des dimensions comprises entre 0,005 et 0,4 μm.

24. Procédé selon la revendication 22, caractérisé en ce que les particules minérales renfermées dans la résine durcissable constituent de 10 à 90 % du poids total du mélange particules/résine.

25. Procédé selon la revendication 1, caractérisé en ce que les particules poreuses minérales subissent un traitement préalable avec un agent intermédiaire conférant une adhérence à base de silane organo-fonctionnel, un mécanisme d'adhérence chimique et un mécanisme de collage avec pénétration et assemblage physiques, en liaison avec une résine organique, à un emprisonnement et une stabilité perfectionnés des particules minérales poreuses.

26. Procédé selon la revendication 25, caractérisé en ce qu'au moins une partie de l'agent d'adhérence à base de silane est ajoutée dans une proportion en poids de 0,05 à 10 % calculée sur le poids de masse totale.

27. Procédé selon la revendication 1, caractérisé en ce que les particules minérales poreuses contiennent une proportion volumique de 30 à 80 % de substance minérale.

28. Procédé selon la revendication 17, caractérisé en ce que la dimension des particules minérales poreuses est inférieure à 300 μm.

29. Procédé selon la revendication 1, caractérisé en ce que les particules minérales poreuses contiennent, dans leur zone extérieure, une résine durcissable et dans leur zone centrale, une résine durcie.

30. Procédé selon la revendication 1, caractérisé en ce que les particules minérales poreuses sont imprégnées d'une résine thermoplastique et que le compactage de la masse s'effectue pendant le chauffage de façon telle que les particules minérales poreuses se collent les unes aux autres.

31. Procédé selon l'une des revendications 1 et 17, caractérisé en ce que la résine durcissable contient essentiellement au moins 1 monomère et/ou polymère choisis dans le groupe constitué par les résines acryliques, le chlorure de vinyle, l'acétate de cellulose, le styrène et les copolymères acrylo-nitriles.

32. Procédé selon la revendication 31, caractérisé en ce que la résine durcissable est constituée par au moins un monomère et/ou polymère acryliques, choisis dans le groupe constitué par les méthacrylate d'éthyle, acrylate d'éthyle et méthacrylate de méthyle.

33. Procédé selon la revendication 31, caractérisé en ce que la résine durcissable est essentiellement constituée par un copolymère formé à partir d'un monomère acrylique et d'un autre monomère copolymérisable.

34. Procédé selon la revendication 31, caractérisé en ce que la résine durcissable est un copolymère formé à partir d'un monomère acrylique et d'un autre monomère copolymérisable, qui est choisi dans le groupe constitué par les styrène, butadiène, éthylène et acide acrylique.

35. Procédé selon la revendication 31, caractérisé en ce que la résine durcissable contient essentiellement du bis-GMA ou un autre dérivé quelconque du bisphénol A.

36. Procédé selon la revendication 1, caractérisé en ce que le durcissement de la résine est obtenu par photoionisation d'une réaction de polymérisation.

37. Procédé selon la revendication 1, caractérisé en ce qu'une résine composite, qui est essentiellement constituée de particules minérales poreuses, qui sont partiellement ou totalement imprégnées par un monomère au moins partiellement polymérisable et/ou un polymère, et qui sont réparties par compactage ou un autre procédé de façon que les particules soient en contact mutuel et se collent ensemble lors de la polymérisation afin de former une structure de résine avec une phase minérale contiguë.

0,01mm